# EUROPÄISCHE PATENTSCHRIFT

(11) **EP 1 968 743 B1**
(45) Veröffentlichungstag und Bekanntmachung des Hinweises auf die Patenterteilung: **23.03.2011**
(21) Anmeldenummer: 06830762.8
(22) Anmeldetag: 20.12.2006
(51) Int. Cl.: B01J 23/68, C07C 51/265, C07C 51/31, B01J 37/08, B01J 37/02, B01J 35/00

(54) **UMWANDLUNG EINES PRÄKATALYSATORS IN EINE KATALYTISCH AKTIVE SILBER-VANADIUMOXID-BRONZE**
CONVERSION OF A PRECATALYST TO A CATALYTICALLY ACTIVE SILVER-VANADIUM OXIDE BRONZE
CONVERSION D'UN PRE-CATALYSEUR EN UN BRONZE D'OXYDE D'ARGENT-VANADIUM CATALYTIQUEMENT ACTIF

(30) Priorität: 21.12.2005 DE 102005061382
(43) Veröffentlichungstag der Anmeldung: 17.09.2008
(73) Patentinhaber: BASF SE, 67056 Ludwigshafen (DE)
(72) Erfinder: NETO, Samuel, 01099 Dresden (DE); HIBST, Hartmut, 69198 Schriesheim (DE); ROSOWSKI, Frank, 68165 Mannheim (DE); STORCK, Sebastian, 68167 Mannheim (DE); ZÜHLKE, Jürgen, 67346 Speyer (DE)
(86) Internationale Anmeldenummer: PCT/EP2006/070041
(87) Internationale Veröffentlichungsnummer: WO 2007/071749

(56) Entgegenhaltungen:
- WO-A-00/27753
- DE-A1- 19 705 326

## Beschreibung

Die Erfindung betrifft ein Verfahren zur Umwandlung eines Multimetalloxid-Präkatalysators in einen Gasphasenoxidationskatalysator mit einer katalytisch aktiven Silber-Vanadiumoxid-Bronze, insbesondere einen Katalysator zur Gasphasenpartialoxidation von aromatischen Kohlenwasserstoffen, zu Aldehyden, Carbonsäuren und/oder Carbonsäureanhydriden.

Eine Vielzahl von Aldehyden, Carbonsäuren und/oder Carbonsäureanhydriden wird technisch durch die katalytische Gasphasenoxidation von aromatischen Kohlenwasserstoffen wie Benzol, o-, m- oder p-Xylol, Naphthalin, Toluol oder Durol (1,2,4,5-Tetramethylbenzol) in Festbettreaktoren hergestellt. Je nach Ausgangsmaterial werden so beispielsweise Benzaldehyd, Benzoesäure, Maleinsäureanhydrid, Phthalsäureanhydrid, Isophthalsäure, Terephthalsäure oder Pyromellithsäureanhydrid gewonnen. Dazu wird ein sauerstoffhaltiges Gas, beispielsweise Luft, und das zu oxidierende Ausgangsmaterial durch eine Vielzahl in einem Reaktor angeordneter Rohre geleitet, in denen sich eine Schüttung mindestens eines Katalysators befindet.

In der WO 00/27753, der WO 01/85337 und der WO 2005/012216 werden Silber- und Vanadiumoxid enthaltende Multimetalloxide beschrieben. Bei der thermischen Behandlung werden die Multimetalloxide in Silber-Vanadiumoxid-Bronzen umgewandelt, die die partielle Oxidation von aromatischen Kohlenwasserstoffen katalysieren. Unter Silber-Vanadiumoxid-Bronzen werden Silber-Vanadiumoxid-Verbindungen mit einem atomaren Ag : V-Verhältnis von weniger als 1 verstanden. Es handelt sich im Allgemeinen um halbleitende oder metallisch leitfähige, oxidische Festkörper, die bevorzugt in Schicht- oder Tunnelstrukturen kristallisieren, wobei das Vanadium im [V₂O₅]-Wirtsgitter teilweise reduziert zu V(IV) vorliegt. Die thermische Umwandlung der Multimetalloxide zu Silber-Vanadiumoxid-Bronzen verläuft über eine Reihe von Reduktions- und Oxidationsreaktionen, die im Einzelnen noch nicht verstanden sind.

In der Praxis wird das Multimetalloxid als Schicht auf einen inerten Träger aufgebracht, wobei man einen so genannten Präkatalysator erhält. Die Umwandlung des Präkatalysators in den aktiven Katalysator erfolgt meist in situ im Oxidationsreaktor unter den Bedingungen der Oxidation von aromatischen Kohlenwasserstoffen zu Aldehyden, Carbonsäuren und/oder Carbonsäureanhydriden. Um eine thermische Schädigung des Katalysators zu vermeiden, muss bei der in situ-Umwandlung die Kohlenwasserstoff-Beladung des Gasstroms mit dem zu oxidierenden Kohlenwasserstoff von sehr niedrigen Werten langsam gesteigert werden, wobei die Hot-Spot-Temperatur in der Katalysatorschüttung kontrolliert wird. Es zieht sich in der Regel über mehrere Tage oder Wochen hin, bis die endgültige Beladung erreicht ist, bei der die produktive Kohlenwasserstoff-Oxidation erfolgt.

Wie dargelegt, ist die in situ-Umwandlung der Präkatalysatoren ein zeitaufwändiger Prozess. Außerdem ist die genaue Dosierung der geringen Kohlenwasserstoffmengen zu Beginn des Prozesses vielfach schwierig. Es ist daher wünschenswert, die Präkatalysatoren außerhalb des Gasphasenoxidationsreaktors geeignet vorzubehandeln, so dass die produktive Gasphasenoxidation unmittelbar nach dem Katalysatoreinbau gestartet werden kann.

Die WO 00/27753 offenbart, dass die Umwandlung des Präkatalysators auch außerhalb des Oxidationsreaktors durch thermische Behandlung bei Temperaturen oberhalb 200 bis 650 °C erfolgen kann, wobei Einflussgrößen, wie die Zusammensetzung der Gasatmosphäre, An- oder Abwesenheit eines Bindemittels sowie Art und Menge eines Bindemittels zu berücksichtigen sind. Die optimalen Bedingungen sollen in einem Vorversuch ermittelt werden. Genauere Angaben zu diesen Bedingungen macht die Druckschrift nicht.

Der Erfindung liegt die Aufgabe zu Grunde, ein zweckmäßiges Verfahren anzugeben, nach dem die Präkatalysatoren außerhalb des Oxidationsreaktors in die aktiven Gasphasenoxidationskatalysatoren umgewandelt werden können.

Die Aufgabe wird erfindungsgemäß gelöst durch ein Verfahren zur Umwandlung eines Präkatalysators, der einen inerten Träger, eine organische Kohlenstoffquelle und ein Silber und Vanadium enthaltendes Multimetalloxid umfasst, in einen Gasphasenoxidationskatalysator, der den inerten Träger und eine katalytisch aktive Silber-Vanadiumoxid-Bronze umfasst, bei dem man den Präkatalysator in einer Gasatmosphäre, die weniger als 10 Vol.-% Sauerstoff enthält, bei einer Temperatur von wenigstens 350 °C thermisch behandelt, wobei man vor der thermischen Behandlung die Menge der Kohlenstoffquelle im Präkatalysator auf einen (von Null verschiedenen) Wert unterhalb einer kritischen Menge einstellt, indem man den Präkatalysator in einer sauerstoffhaltigen Atmosphäre bei einer Temperatur von 80 bis 200°C behandelt und die kritische Menge als die Menge an Kohlenstoffquelle definiert ist, ab der bei der thermischen Behandlung des Präkatalysators eine Reduktion zu elementarem Silber erfolgt.

Im Ausgangs-Multimetalloxid liegt das Vanadium in der Oxidationsstufe 5 (Vanadium(V)) vor; in der Silber-Vanadiumoxid-Bronze beträgt die durchschnittliche Vanadiumoxidationsstufe 4,5 bis 4,7.

Die nach der erfindungsgemäßen thermischen Behandlung erhaltenen Katalysatoren zeigen eine ausreichende Abriebfestigkeit und können problemlos gehandhabt, transportiert und in Reaktionsrohre eingefüllt werden.

Die Gasatmosphäre, in der die thermische Behandlung erfolgt, enthält weniger als 10 Vol.-%, vorzugsweise weniger als 3 Vol.-% und insbesondere weniger als 1 Vol.-% (molekularen) Sauerstoff. In der Regel verwendet man ein Inertgas, vorzugsweise Stickstoff, das im Wesentlichen sauerstofffrei ist. Zweckmäßigerweise wird die thermische Behandlung in einem Gasstrom, vorzugsweise einem Inertgasstrom, durchführt.

Die thermische Behandlung kann in allen geeigneten Apparaten durchgeführt werden, z. B. in Hordenöfen, Drehkugelöfen, beheizbaren Reaktoren, in denen eine Schüttung des Präkatalysators vom Gasstrom durchströmt wird, und dergleichen. Die thermische Behandlung erfolgt bei einer Temperatur von wenigstens 350 °C, vorzugsweise wenigstens 400 °C, insbesondere 400 bis 600 °C. Höhere Temperaturen innerhalb des angegebenen Bereichs führen üblicherweise zu einer höheren Kristallinität und einer geringeren BET-Oberfläche der Silber-Vanadiumoxid-Bronze. Die Aufheizrate ist nicht besonders kritisch, 1 bis 10 °C/min sind im Allgemeinen geeignet. Die Dauer der thermischen Behandlung beträgt im Allgemeinen 0,5 bis 12 Stunden, vorzugsweise 1 bis 5 Stunden.

Der Präkatalysator enthält eine organische Kohlenstoffquelle. Die Kohlenstoffquelle dient bei der thermischen Behandlung des Präkatalysators vermutlich als Reduktionsmittel für eine teilweise Reduktion des im Multimetalloxid enthaltenen Vanadium(V) zu V(IV) vorliegt.

Als Kohlenstoffquellen eignen sich typische Hilfsstoffe, die bei der Herstellung der Präkatalysatoren z. B. als Porenbildner oder Bindemittel verwendet werden. Im Allgemeinen handelt es sich um (i) Verbindungen mit 2 bis 12 Kohlenstoffatomen, die wenigstens eine funktionelle Gruppe aufweisen, die ausgewählt ist unter OH, C=O und NH₂; und/oder (ii) polymere Verbindungen, die aufgebaut sind aus Wiederholungseinheiten mit 2 bis 12 Kohlenstoffatomen, die wenigstens eine funktionelle Gruppe aufweisen, die ausgewählt ist unter OH, C=O und NH₂. Die Ketogruppe (C=O) kann auch Bestandteil einer Carboxamid-, Carbonsäure-, Carbonsäureester- oder Carbonsäureanhydridgruppe sein. Bevorzugt ist die Kohlenstoffquelle ausgewählt ist unter Verbindungen mit 2 bis 6 Kohlenstoffatomen, die wenigstens zwei funktionelle Gruppen aufweisen, die unabhängig voneinander ausgewählt sind unter OH, C=O und NH₂.

Zu den geeigneten Kohlenstoffquellen zählen beispielsweise Ethylenglycol, Propylenglycol, Glycerin, Pentaerythrit, Pentosen, Hexosen, Oxalsäure, Ammoniumoxalat, Malonsäure, Maleinsäure, Fumarsäure, Bernsteinsäure, Ascorbinsäure, Benzoesäure, o-, m- und p-Tolylsäure, Phthalsäure, Phthalsäureanhydrid, Isophthalsäure, Terephthalsäure, Dimethylformamid, Dimethylacetamid, N-Methylpyrrolidon;

Zu den geeigneten Kohlenstoffquellen zählen weiterhin Polymere wie Polyalkylenglycole, Polyalkylenamine, Polysaccharide, Polyvinylalkohol, Vinylacetat/Vinyllaurat-, Vinylacetat/Acrylat-, Styrol/Acrylat-, Vinylacetat/Maleat- oder Vinylacetat/EthylenCopolymere.

Es wurde gefunden, dass die Menge der Kohlenstoffquelle im Präkatalysator kontrolliert werden muss. Ist die Menge der Kohlenstoffquelle zu hoch, wird bei der thermischen Behandlung des Präkatalysators keine Silber-Vanadiumoxid-Bronze gebildet, sondern die im Multimetalloxid enthaltenen Silberionen werden zu elementarem Silber reduziert. Während die Silber-Vanadiumoxid-Bronze eine dunkelgrüne Farbe aufweist, erscheint das auf dem Katalysator abgeschiedene elementare Silber schwarz. Das Vorhandensein von elementarem Silber kann auch im Pulverröntgendiffraktogramm am Auftreten von Beugungsreflexen festgestellt werden, die dem kubischen Silbergitter zuzuordnen sind. Überraschenderweise findet die Reduktion zu elementarem Silber ab einem Grenzwert der Menge der Kohlenstoffquelle schlagartig statt. Der Grenzwert wird für die Zwecke der vorliegenden Patentanmeldung als "kritische Menge an Kohlenstoffquelle" bezeichnet.

Die kritische Menge hängt von der chemischen Natur der Kohlenstoffquelle ab. Sie kann vom Fachmann in Vorversuchen leicht ermittelt werden. Z. B. kann eine Probemenge eines Präkatalysator mit einem gegebenen Gehalt an Kohlenstoffquelle der thermischen Behandlung (z. B. 4 Stunden bei 490 °C im Stickstoffstrom) unterzogen werden, und der erhaltene Katalysator auf das Auftreten von elementarem Silber untersucht werden. Erfolgte eine Reduktion zu elementarem Silber, so kann der Fachmann (in einer frischen Probemenge des Präkatalysators) den Kohlenstoffgehalt (gemäß dem nachstehend beschriebenen Verfahren) schrittweise senken und den Präkatalysator erneut einer thermischen Behandlung unterwerfen. Auf diese Weise kann die kritische Menge ohne Weiteres anhand weniger Versuche rasch eingegrenzt werden.

Vorzugsweise stellt man die Menge der Kohlenstoffquelle im Präkatalysator vor der thermischen Behandlung auf einen Wert von weniger als 2 Gew.-% (gerechnet als Kohlenstoff bezogen auf das Gewicht des Multimetalloxids) ein, z. B. einen Wert im Bereich von 0,5 bis weniger als 2 Gew.-%, besonders bevorzugt auf einen Wert von weniger als oder gleich 1,3 Gew.-%. Die Menge der Kohlenstoffquelle im Präkatalysator vor der thermischen Behandlung beträgt im Allgemeinen wenigstens 0,1 Gew.-%, meist wenigstens 0,5 Gew.-%, bezogen auf das Gewicht des Multimetalloxids.

Der Kohlenstoffgehalt kann ermittelt werden, indem man eine genau eingewogene Probe der Aktivmasse des (Prä)katalysators im Sauerstoffstrom verbrennt und das gebildete Kohlendioxid quantitativ detektiert, z. B. mittels einer IR-Zelle.

Um den Gehalt der Kohlenstoffquelle geeignet einzustellen, kann der Fachmann bei der Herstellung des Präkatalysators konsequent Porenbilder, Bindemittel und weitere Hilfsstoffe mit geringem Kohlenstoffgehalt auswählen bzw. kohlenstoffhaltige Hilfsstoffe nur in untergeordneten Mengen verwenden. Im Allgemeinen ist es aber im Hinblick auf eine vernünftige Haftung des Multimetalloxids auf dem Träger, eine gewünschte Porenstruktur und andere Faktoren unerlässlich, bei der Präkatalysatorherstellung größere Mengen kohlenstoffhaltiger Hilfsstoffe zu verwenden.

In den meisten Fällen enthält der Präkatalysator daher anfangs eine Menge an Kohlenstoffquelle, die größer ist als die kritische Menge oder dieser entspricht. Meist enthält der unbehandelte Präkatalysator Mengen an Kohlenstoffquellen, die 3 bis 10 Gew.-% Kohlenstoff, bezogen auf das Gewicht des Multimetalloxids, entsprechen. Die Menge an Kohlenstoffquelle wird auf einen Wert unterhalb der kritischen Menge eingestellt, indem man den Präkatalysator in einer sauerstoffhaltigen Atmosphäre bei einer Temperatur von 80 bis 200 °C tempert bzw. abbrennt. Unter "Abbrennen" soll eine Verringerung des Kohlenstoffgehalts verstanden werden, wobei ein Teil der Kohlenstoffquelle verdampft, absublimiert und/oder oxidativ zu gasförmigen Produkten wie Kohlendioxid zersetzt wird.

Das Abbrennen kann in allen geeigneten Apparaten durchgeführt werden, z. B. solchen, wie sie für die anschließende thermische Behandlung des Präkatalysators verwendet werden. Um eine allzu rasche Zersetzung der Kohlenstoffquelle mit stark exothermer Wärmetönung und eine potentielle thermische Schädigung des Katalysators zu vermeiden, umfasst das Abbrennen vorzugsweise wenigstens eine Aufheizphase, während welcher man die Temperatur des Präkatalysators mit einer Rate von weniger als 5 °C/min (insbesondere weniger als 1,5 °C/min) erhöht, und wenigstens eine Plateauphase, während welcher man die Temperatur des Präkatalystors im Wesentlichen konstant hält.

Das Abbrennen erfolgt in einer sauerstoffhaltigen Atmosphäre; die Atmosphäre enthält vorzugsweise wenigstens 5 Vol-%, z.B. wenigstens 12,5 Vol.-%, und bis zu 25 Vol.-% (molekularen) Sauerstoff. Geeigneterweise verwendet man Luft. Besonders bevorzugt führt man das Abbrennen in einem Luftstrom durch. Das Abbrennen erfolgt bei einer Temperatur von 80 bis 200 °C, vorzugsweise 120 bis 190 °C.

Geeignete Multimetalloxide, ihre Herstellung und ihre Aufbringung auf inerte Träger sind an sich bekannt und z. B. in der WO 00/27753, WO 01/85337 und WO 2005/012216 beschrieben.

Im Allgemeinen weist das Multimetalloxid die allgemeine Formel I auf:

Ag_{a-c}Q_{b}M_{c}V₂O_{d} * e H₂O, I

worin
- a: einen Wert von 0,3 bis 1,9 hat, vorzugsweise 0,5 bis 1,0 und besonders bevor- zugt 0,6 bis 0,9;
- Q: für ein unter P, As, Sb und/oder Bi ausgewähltes Element steht,
- b: einen Wert von 0 bis 0,3 hat, vorzugsweise 0 bis 0,1
- M: für wenigstens ein unter Alkali- und Erdalkalimetallen, Bi, TI, Cu, Zn, Cd, Pb, Cr, Au, Al, Fe, Co, Ni, Mo, Nb, Ce, W, Mn, Ta, Pd, Pt, Ru und/oder Rh ausgewähltes Metall steht, vorzugsweise Nb, Ce, W, Mn und Ta, insbesondere Ce und Mn, wo- von Ce am meisten bevorzugt ist
- c: einen Wert von 0 bis 0,5 hat, vorzugsweise 0,005 bis 0,2, insbesondere 0;01 bis 0,1; mit der Maßgabe, dass (a-c) ≥ 0,1 ist,
- d: eine Zahl, die sich durch die Wertigkeit und Häufigkeit der von Sauerstoff ver- schiedenen Elemente in der Formel I bestimmt, bedeutet und
- e: einen Wert von 0 bis 20 hat, vorzugsweise 0 bis 5.

Vorzugsweise liegt das Multimetalloxid in einer Kristallstruktur vor, deren Pulverröntgendiagramm durch Beugungsreflexe bei den Netzebenenabständen d 15,23 ± 0,6, 12,16 ± 0,4, 10,68 ± 0,3, 3,41 ± 0,04, 3,09 ±0,04, 3,02 ± 0,04, 2,36 ± 0,04 und 1,80 ± 0,04 Å gekennzeichnet ist.

In der Regel weist das vollständige Pulverröntgenbeugungsdiagramm des Multimetalloxids der Formel I unter anderem die in Tabelle 1 aufgelisteten 17 Beugungsreflexe auf. Weniger intensive Beugungsreflexe des Pulverröntgendiagramms der Multimetalloxide der Formel I wurden in Tabelle 1 nicht berücksichtigt.

**Tabelle 1:**

| Beugungsreflex | d | Iᵣₑₗ (%) |
|---|---|---|
| 1 | 15,23 ± 0,6 | 16 |
| 2 | 12,16±0,4 | 11 |
| 3 | 10,68±0,3 | 18 |
| 4 | 5,06 ± 0,06 | 11 |
| 5 | 4,37 ± 0,04 | 23 |
| 6 | 3,86 ± 0,04 | 16 |
| 7 | 3,41 ± 0,04 | 80 |
| 8 | 3,09 ± 0,04 | 61 |
| 9 | 3,02 ± 0,04 | 100 |
| 10 | 2,58 ± 0,04 | 23 |
| 11 | 2,48 ± 0,04 | 24 |
| 12 | 2,42 ± 0,04 | 23 |
| 13 | 2,36 ± 0,04 | 38 |
| 14 | 2,04 ± 0,04 | 26 |
| 15 | 1,93 ± 0,04 | 31 |
| 16 | 1,80 ± 0,04 | 43 |
| 17 | 1,55 ± 0,04 | 36 |

Die Angabe der Röntgenbeugungsreflexe erfolgt in dieser Anmeldung in Form der von der Wellenlänge der verwendeten Röntgenstrahlung unabhängigen Netzebenenabstände d[Å], die sich aus dem gemessenen Beugungswinkel mittels der Bragg'schen Gleichung errechnen lassen.

Die spezifische Oberfläche nach BET, gemessen gemäß DIN 66 131, die auf den "Recommendations 1984" der IUPAC International Union of Pure and Applied Chemistry (s. Pure & Appl. Chem. 57, 603 (1985)) basiert, beträgt in der Regel mehr als 1 m²/g, bevorzugt 3 bis 250 m²/g, insbesondere 10 bis 250 m²/g und besonders bevorzugt 20 bis 80 m²/g_{.}

Zur Herstellung der Multimetalloxide wird im Allgemeinen eine Suspension von Vanadiumpentoxid (V₂O₅) mit der Lösung einer Silberverbindung und einer Lösung einer Verbindung der Metallkomponente M sowie gegebenenfalls der Lösung einer Verbindung von Q erhitzt. Als Lösungsmittel für diese Umsetzung können polare organische Lösungsmittel dienen, bevorzugt wird als Lösungsmittel Wasser verwendet. Als Silbersalz wird bevorzugt Silbernitrat verwendet.

Sofern mitverwendet, können das oder die Elemente Q aus der Gruppe P, As, Sb und/oder Bi in elementarer Form oder als Oxide oder Hydroxide eingesetzt werden. Vorzugsweise werden ihre teilneutralisierten oder freien Säuren verwendet, wie Phosphorsäure, Arsenwasserstoffsäure, Antimonwasserstoffsäure, die Ammoniumhydrogenphosphate, -arsenate, -antimonate und -bismutate und die Alkalihydrogenphosphate, -arsenate, -antimonate und -bismutate. Ganz besonders bevorzugt verwendet man als Element Q Phosphor allein, insbesondere in Form von Phosphorsäure, phosphoriger Säure, hypophosphoriger Säure, Ammoniumphosphat oder Phosphorsäureester und vor allem als Ammoniumdihydrogenphosphat.

Als Salze der Metallkomponente M werden in der Regel wasserlösliche Salze verwendet, beispielsweise die Perchlorate oder Carboxylate, insbesondere die Acetate. Bevorzugt werden die Nitrate der betreffenden Metallkomponente M verwendet, insbesondere Cernitrat oder Mangannitrat.

Die Umsetzung des V₂O₅ mit der Silberverbindung, der Verbindung der Metallkomponente M und gegebenenfalls Q kann im Allgemeinen bei Raumtemperatur oder bei erhöhter Temperatur durchgeführt werden. In der Regel wird die Umsetzung bei Temperaturen von 20 bis 375 °C, vorzugsweise bei 20 bis 100 °C und besonders bevorzugt bei 60 bis 100 °C vorgenommen. Liegt die Temperatur der Umsetzung oberhalb der Temperatur des Siedepunktes des verwendeten Lösungsmittels, wird die Umsetzung zweckmäßigerweise unter dem Eigendruck des Reaktionssystems in einem Druckgefäß ausgeführt. Vorzugsweise werden die Reaktionsbedingungen so gewählt, dass die Umsetzung bei Atmosphärendruck durchgeführt werden kann. Die Dauer dieser Umsetzung kann in Abhängigkeit von der Art der umgesetzten Ausgangsmaterialien und den angewandten Temperaturbedingungen 10 Minuten bis 3 Tage betragen. Eine Verlängerung der Reaktionszeit der Umsetzung, beispielsweise auf 5 Tage und mehr, ist möglich. In der Regel wird die Umsetzung des V₂O₅ mit der Silberverbindung, der Verbindung der Metallkomponente M zum Multimetalloxid während eines Zeitraums von 6 bis 24 Stunden durchgeführt. Bei der Umsetzung verändert sich die orangerote Farbe der V₂O₅-Suspension und es bildet sich die neue Verbindung in Form einer dunkelbraunen Suspension.

Das so gebildete Multimetalloxid kann aus der Reaktionsmischung isoliert werden. Besonders vorteilhaft wird die erhaltene Multimetalloxid-Suspension sprühgetrocknet. Die Sprühtrocknung wird im Allgemeinen unter Atmosphärendruck oder vermindertem Druck vorgenommen. Je nach angewandtem Druck und verwendetem Lösungsmittel bestimmt sich die Eingangstemperatur des Trocknungsgases - im Allgemeinen wird als solches Luft verwendet, es können aber selbstverständlich auch andere Trocknungsgase wie Stickstoff oder Argon, benutzt werden. Die Eingangstemperatur des Trocknungsgases in den Sprühtrockner wird vorteilhaft so gewählt, dass die Ausgangstemperatur des durch Verdampfung des Lösungsmittels abgekühlten Trocknungsgases 200 °C für einen längeren Zeitraum nicht übersteigt. In der Regel wird die Ausgangstemperatur des Trocknungsgases auf 50 bis 150 °C, vorzugsweise 100 bis 140 °C eingestellt.

Bei dem Präkatalysatorhandelt es sich um eine Vorstufe des Katalysators, der aus einem inerten Trägermaterial und wenigstens einer darauf schalenförmig aufgebrachten Schicht besteht, wobei diese Schicht vorzugsweise 30 bis 100 Gew.-%, insbesondere 50 bis 100 Gew.-%, bezogen auf das Gesamtgewicht dieser Schicht, eines Multimetalloxids gemäß Formel I enthalten. Besonders bevorzugt besteht die Schicht vollständig aus einem Multimetalloxid gemäß Formel I. Enthält die katalytisch aktive Schicht außer dem Multimetalloxid gemäß Formel I noch weitere Komponenten, können dies z. B. Inertmaterialien, wie Siliciumcarbid oder Steatit, oder aber auch sonstige bekannte Katalysatoren zur Oxidation von aromatischen Kohlenwasserstoffen zu Aldehyden, Carbonsäuren und/oder Carbonsäureanhydriden auf Vanadiumoxid/Anatas-Basis sein. Vorzugsweise enthält der Präkatalysator 5 bis 25 Gew.-%, bezogen auf das Gesamtgewicht des Präkatalysators, Multimetalloxid.

Als inertes Trägermaterial für die Präkatalysatoren und Schalenkatalysatoren können praktisch alle Trägermaterialien des Standes der Technik, wie sie vorteilhaft bei der Herstellung von Schalenkatalysatoren für die Oxidation aromatischer Kohlenwasserstoffe zu Aldehyden, Carbonsäuren und/oder Carbonsäureanhydriden eingesetzt werden, Verwendung finden, beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Der Träger ist im Allgemeinen "nicht-porös". Der Begriff ist dabei im Sinne von "bis auf technisch unwirksame Mengen an Poren nicht-porös" zu verstehen, da technisch unvermeidlich eine geringe Anzahl Poren im Trägermaterial, das idealerweise keine Poren enthalten sollte, vorhanden sein können. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Wie erwähnt, können die vorstehend genannten Trägermaterialien in Pulverform auch der katalytisch aktiven Masse der erfindungsgemäßen Schalenkatalysatoren zugemischt werden.

Zur schalenförmigen Beschichtung des inerten Trägermaterials mit dem Multimetalloxid können bekannte Methoden angewandt werden. Beispielsweise kann eine Aufschlämmung des nach Isolierung und Trocknung erhaltenen Pulvers des Multimetalloxids in einer beheizten Dragiertrommel auf den Katalysatorträger aufgesprüht werden. Es können auch Wirbelbettbeschichter zur schalenförmigen Aufbringung des Multimetalloxids auf den Katalysatorträger eingesetzt werden. Die Suspension des Multimetalloxids kann in Wasser, einem organischen Lösungsmittel, wie höheren Alkoholen, mehrwertigen Alkoholen, z. B. Ethylenglykol, 1,4-Butandiol oder Glycerin_{;} Dimethylformamid, Dimethylacetamid, Dimethylsulfoxid, N-Methylpyrrolidon oder cyclischen Harnstoffen, wie N,N'-Dimethylethylenharnstoff oder N,N'-Dimethylpropylenharnstoff, oder in Mischungen dieser organischen Lösungsmittel mit Wasser, zubereitet werden. Man kann organische Bindemittel, bevorzugt Copolymere, gelöst oder vorteilhaft in Form einer wässrigen Dispersion zusetzen, wobei im Allgemeinen Bindemittelgehalte von 10 bis 20 Gew.-%, bezogen auf den Feststoffgehalt der Suspension oder Aufschlämmung des erfindungsgemäßen Multimetalloxids angewandt werden. Geeignete Bindemittel sind z. B. Vinylacetat/Vinyllaurat-, Vinylacetat/Acrylat-, Styrol/Acrylat-, Vinylacetat/Maleat- oder Vinylacetat/Ethylen-Copolymere.

Die Schalenkatalysatoren werden für die partielle Oxidation von aromatischen Kohlenwasserstoffen zu Aldehyden, Carbonsäuren und/oder Carbonsäureanhydriden, insbesondere zur Gasphasenpartialoxidation von o-Xylol und/oder Naphthalin zu Phthalsäureanhydrid oder von Toluol zu Benzoesäure und/oder Benzaldehyd, mit einem molekularen Sauerstoff enthaltenden Gas verwendet. Die Katalysatoren können zu diesem Zweck alleine oder in Kombination mit anderen, unterschiedlich aktiven Katalysatoren, beispielsweise Katalysatoren des Standes der Technik auf Vanadiumoxid/Anatas-Basis, eingesetzt werden, wobei die unterschiedlichen Katalysatoren im Allgemeinen in separaten Katalysatorschüttungen, die in einem oder mehreren Katalysatorfestbetten angeordnet sein können, im Reaktor angeordnet werden.

Die Schalenkatalysatoren oder Präkatalysatoren werden hierzu in die Reaktionsrohre eines Röhrenreaktors gefüllt, die von außen, z. B. mittels einer Salzschmelze, auf die Reaktionstemperatur thermostatisiert werden. Über die so bereitete Katalysatorschüttung wird das Reaktionsgas bei Temperaturen von 100 bis 650 °C und vorzugsweise 250 bis 480 °C und bei einem Überdruck von im Allgemeinen 0,1 bis 2,5 bar, vorzugsweise von 0,3 bis 1,5 bar mit einer Raumgeschwindigkeit von im Allgemeinen 750 bis 5000 h⁻¹ geleitet.

Das dem Katalysator zugeführte Reaktionsgas wird im Allgemeinen durch Vermischen von einem molekularen Sauerstoff enthaltenden Gas, das außer Sauerstoff noch geeignete Reaktionsmoderatoren und/oder Verdünnungsmittel, wie Wasserdampf, Kohlendioxid und/oder Stickstoff, enthalten kann, mit dem zu oxidierenden, aromatischen Kohlenwasserstoff erzeugt, wobei das molekularen Sauerstoff enthaltende Gas im Allgemeinen 1 bis 100 Vol.-%, vorzugsweise 2 bis 50 Vol.-% und besonders bevorzugt 10 bis 30 Vol.-% Sauerstoff, 0 bis 30 Vol.-%, vorzugsweise 0 bis 20 Vol.-% Wasserdampf sowie 0 bis 50 Vol.-%, vorzugsweise 0 bis 1 Vol.-% Kohlendioxid, Rest Stickstoff, enthalten kann. Zur Erzeugung des Reaktionsgases wird das molekularen Sauerstoff enthaltende Gas im Allgemeinen mit 30 bis 300 g je Nm³, bevorzugt mit 70 bis 150 g je Nm³ Gas des zu oxidierenden aromatischen Kohlenwasserstoffs beschickt. Besonders vorteilhaft wird als molekularen Sauerstoff enthaltendes Gas Luft verwend et.

Bei einer bevorzugten Ausführungsform des Verfahrens zur partiellen Oxidation von aromatischen Kohlenwasserstoffen zu Aldehyden, Carbonsäuren und/oder Carbonsäureanhydriden, die sich besonders vorteilhaft für die Herstellung von Phthalsäureanhydrid aus o-Xylol und/oder Naphthalin erweist, wird der aromatische Kohlenwasserstoff zunächst an einer Schüttung des erfindungsgemäßen Schalenkatalysators unter Teilumsatz zu einem Reaktionsgemisch umgesetzt. Das erhaltene Reaktionsgemisch oder eine Fraktion davon kann man dann mit wenigstens einem weiteren Katalysator in Kontakt bringen, dessen katalytisch aktive Masse Vanadiumpentoxid und Anatas enthält.

Vorzugsweise leitet man den gasförmigen Strom nacheinander über ein Bett eines stromaufwärts gelegenen Katalysators und ein Bett eines stromabwärts gelegenen Katalysators leitet, wobei das Bett des stromaufwärts gelegenen Katalysators einen erfindungsgemäßen Katalysator enthält und das Bett des stromabwärts gelegenen Katalysators wenigstens einen Katalysator enthält, dessen katalytisch aktive Masse Vanadiumpentoxid und Anatas enthält. Im Allgemeinen enthält die katalytisch aktive Masse des stromabwärts gelegenen Katalysators 1 bis 40 Gew.-% Vanadiumoxid, berechnet als V₂O₅, 60 bis 99 Gew.-% Titandioxid, berechnet als TiO₂, bis zu 1 Gew.-% einer Cäsiumverbindung, berechnet als Cs, bis zu 1 Gew.-% einer Phosphorverbindung, berechnet als P, und bis zu 10 Gew.-% Antimonoxid, berechnet als Sb₂O₃. Mit Vorteil umfasst das Bett des stromabwärts gelegenen Katalysators wenigstens zwei Lagen von Katalysatoren, deren katalytisch aktive Masse unterschiedlichen Cs-Gehalt aufweist, wobei der Cs-Gehalt im Strömungsrichtung des gasförmigen Stroms abnimmt.

Die Erfindung wird durch die folgenden Beispiele und Vergleichsbeispiele näher veranschaulicht.

### Beispiele

### Beispiel 1

In 7 l vollentsalztes Wasser von 60 °C wurden 102 g V₂O₅ (= 0,56 Mol) unter Rühren zugegeben. Die Suspension wurde mit eine wässrigen Lösung von 4,94 g CeNO₃ * 6 H₂O (= 0,011 Mol, Aldrich, Reinheit 99 %) versetzt. In die erhaltene orangefarbene Suspension wurde unter weiterem Rühren eine wässrige Lösung von 68 g AgNO₃ (= 0,398 Mol) in 1 I Wasser zugegeben. Anschließend wurde die Temperatur der erhaltenen Suspension innerhalb von 2 Stunden auf 90 °C erhöht und bei dieser Temperatur die Mischung 24 Stunden gerührt. Danach wurde die erhaltene dunkelbraune Suspension abgekühlt und sprühgetrocknet (Eingangstemperatur (Luft) = 350 °C, Ausgangstemperatur (Luft) = 110 °C). Das Pulver hatte die Zusammensetzung Ce_{0,02}Ag_{0,71}V₂Oₓ.

Das erhaltene Pulver hatte eine spezifische Oberfläche nach BET von 61 m²/g. Vom erhaltenen Pulver wurde ein Pulverröntgendiagramm mit Hilfe eines Diffraktometers D 5000 der Firma Siemens unter Anwendung von Cu-Kα-Strahlung (40 kV, 30 mA) aufgenommen. Das Diffraktometer war mit einem automatischen Primär- und Sekundärblendensystem sowie einm Sekundär-Monochromator und Szintillationsdetektor ausgestattet. Aus dem Pulverröntgendiagramm wurden die folgenden Netzebenenabstände d [Å] mit den dazugehörigen relativen Intensitäten Iᵣₑₗ [%] erhalten: 15,04 (11,9), 11,99 (8,5), 10,66 (15,1), 5,05 (12,5), 4,35 (23), 3,85 (16,9), 3,41 (62,6), 3,09 (55,1), 3,02 (100), 2,58 (23,8), 2,48 (27,7), 2,42 (25,1), 2,36 (34,2), 2,04 (26,4), 1,93 (33,2), 1,80 (35,1), 1,55 (37,8).

Das Pulver wie folgt auf Magnesiumsilikat-Ringe aufgebracht: 350 g Steatit-Ringe mit einem äußeren Durchmesser von 7 mm, einer Länge von 3 mm und einer Wandstärke von 1,5 mm wurden in einer Dragiertrommel bei 20 °C während 20 min mit 85 g des Pulvers und 8,5 g Oxalsäure unter Zusatz von 50 ml einer 12,5 Gew.-% wässerigen Glycerin-Lösung beschichtet und anschließend getrocknet. Das Gewicht der so aufgetragenen katalytisch aktiven Masse, bestimmt an einer Probe des erhaltenen Präkatalysators, betrug nach einstündiger Wärmebehandlung bei 450 °C 18 Gew.-%, bezogen auf das Gesamtgewicht des fertigen Katalysators. Der Kohlenstoffgehalt betrug etwa 4 Ge.-% (bezogen auf die Aktivmasse).

Nach der Beschichtung wurde der Präkatalysator in einem Umluftofen unter einer LuftAtmosphäre mit einer Aufheizrate von 0,33 °C/min auf 140 °C erwärmt und 4 Stunden bei dieser Temperatur gehalten. Nach dieser Behandlung betrug der Kohlenstoffanteil im Präkatalysator etwa 1 Gew.-% (bezogen auf die Aktivmasse). Danach wurde der Präkatalysator unter in einem Drehkugelofen (Kugel von 500 ml) unter einer N₂-Atmosphäre (1 NI/h-g_{Aktivmasse} N₂) mit einer Aufheizrate von 2 °C/min auf 490 °C erhitzt und 4 Stunden bei dieser Temperatur gehalten.

Nach dieser Behandlung erschien die Aktivmasse dunkelgrün; der Kohlenstoffgehalt betrug 0,007 Gew.-% (bezogen auf die Aktivmasse). Mittels Röntgendiffraktometrie wurde gezeigt, dass es sich um eine kristalline δ-Bronze handelte. Die BET-Oberfläche betrug 3,9 m²/g, die durchschnittliche Vanadiumoxidationsstufe 4,67.

Nach dem Einbau des so vorbereiteten Katalysators in einen Oxidationsreaktor zusammen mit zwei nachgeordneten Schichten unterschiedlich aktiver V₂O₅/TiO₂-Katalysatoren konnte die Gasphasenoxidation von o-Xylol zu Phthalsäureanhydrid mit einer Anfangsbeladung von etwa 30 g/Nm³ Luft gestartet werden, die zügig auf etwa 80 g/Nm³ gesteigert werden konnte.

Zum Vergleich wies eine Ausbauprobe eines in situ hergestellten Katalysators einen Kohlenstoffgehalt 0,005 Gew.-% auf (bezogen auf die Aktivmasse). Laut Röntgendiffraktometrie handelte es sich bei der Ausbauprobe um eine kristalline δ-Bronze (Aus dem Pulverröntgendiagramm wurden die folgenden Netzebenenabstände d [Å] mit den dazugehörigen relativen Intensitäten Iᵣₑₗ [%] erhalten: 4,85 (9,8), 3,50 (14,8), 3,25 (39,9), 2,93 (100), 2,78 (36,2), 2,55 (35,3), 2,43 (18,6), 1,97 (15,2), 1,95 (28,1), 1,86 (16,5), 1,83 (37,5), 1,52 (23,5).). Die BET-Oberfläche betrug 6,7 m²/g, die durchschnittliche Vanadiumoxidationsstufe 4,63.

### Vergleichsbeispiel 2

Beispiel 1 wurde wiederholt, wobei jedoch der Präkatalysator im ersten Behandlungsschritt nur 2 Stunden auf 100 °C an der Luft erwärmt wurde. Der Kohlenstoffgehalt im Präkatalysator betrug 2,6 Gew.-%. Danach wurde der Präkatalysator 4 Stunden im Stickstoffstrom auf 450 °C gehalten.

Der erhaltene Katalysator war schwarz; im Pulverdiffraktogramm war ein einziger intensiver Peak zu erkennen, der dem kubischem Gitter von Silber zuzuordnen ist. Es wurden keine Peaks beobachtet, die der δ-Bronze zuzuordnen sind. Die BET-Oberfläche betrug 30 m²/g, die durchschnittliche Vanadiumoxidationsstufe 4,1-4,2 (der Katalysator war überreduziert). Der Kohlenstoffgehalt betrug weniger als 0,02 Gew.-%.

### Vergleichsbeispiel 3

Vergleichsbeispiel 2 wurde wiederholt; jedoch erfolgte die thermische Behandlung bei 450 °C im Luftstrom.

Die Aktivmasse des erhaltenen Katalysators umfasste gemäß Röntgendiffraktogramm ein Gemisch von β-Ag_{0,33}V₂O₅ und Ag_{1,2}V₃O₈. Die durchschnittliche Vanadiumoxidationsstufe betrug 4,8.

## Patentansprüche

1. Verfahren zur Umwandlung eines Präkatalysators, der einen inerten Träger, eine organische Kohlenstoffquelle und ein Silber und Vanadium enthaltendes Multimetalloxid umfasst, in einen Gasphasenoxidationskatalysator, der den inerten Träger und eine katalytisch aktive Silber-Vanadiumoxid-Bronze umfasst, worin die durchschnittliche Vanadium-Oxidationsstufe 4,5 bis 4,7 beträgt, wobei der Präkatalysator anfangs eine Menge an Kohlenstoffquelle enthält, die größer ist als eine kritische Menge oder dieser entspricht, wobei man
(i) die Menge an Kohlenstoffquelle durch Abbrennen auf einen Wert unterhalb der kritischen Menge einstellt, indem man den Präkatalysator in einer sauerstoffhaltigen Atmosphäre bei einer Temperatur von 80 bis 200 °C behandelt; und danach
(ii) den Präkatalysator in einer Gasatmosphäre, die weniger als 10 Vol.-% Sauerstoff enthält, bei einer Temperatur von wenigstens 350 °C thermisch behandelt,
wobei die kritische Menge als die Menge an Kohlenstoffquelle definiert ist, ab der bei der thermischen Behandlung des Präkatalysators eine Reduktion zu elementarem Silber erfolgt.

2. Verfahren nach Anspruch 1, wobei man die thermische Behandlung in einem inertgasstrom durchführt.

3. Verfahren nach Anspruch 1 oder 2, wobei man vor der thermischen Behandlung die Menge der Kohlenstoffquelle im Präkatalysator auf einen Wert im Bereich von 0,5 bis weniger als 2 Gew.-%, gerechnet als Kohlenstoff bezogen auf das Gewicht des Multimetalloxids, einstellt.

4. Verfahren nach Anspruch 3, wobei man vor der thermischen Behandlung die Menge der Kohlenstoffquelle im Präkatalysator auf einen Wert von weniger als oder gleich 1,3 Gew.-% einstellt.

5. Verfahren nach einem der vorhergehenden Ansprüche, wobei das Abbrennen wenigstens eine Aufheizphase, während welcher man die Temperatur des Präkatalysators mit einer Rate von weniger als 5 °C/min erhöht, und wenigstens eine Plateauphase, während welcher man die Temperatur des Präkatalysators im Wesentlichen konstant hält, umfasst. -

6. Verfahren nach einem der vorhergehenden Ansprüche, wobei man das Abbrennen in einem Luftstrom durchführt.

7. Verfahren nach einem der vorhergehenden Ansprüche, wobei die Kohlenstoffquelle ausgewählt ist unter (i) Verbindungen mit 1 bis 12 Kohlenstoffatomen, die wenigstens eine funktionelle Gruppe aufweisen, die ausgewählt ist unter OH, C=O und NH₂; und (ii) polymeren Verbindungen. die aufgebaut sind aus Wiederholungseinheiten mit 2 bis 12 Kohlenstoffatomen, die wenigstens eine funktionelle Gruppe aufweisen, die ausgewählt ist unter OH, C=O und NH₂.

8. Verfahren nach Anspruch 7, wobei die Kohlenstoffquelle ausgewählt ist unter Verbindungen mit 2 bis 6 Kohlenstoffatomen, die wenigstens zwei funktionelle Gruppen aufweisen, die unabhängig voneinander ausgewählt sind unter OH, C=O und NH₂.

9. Verfahren nach Anspruch 7, wobei die Kohlenstoffquelle ausgewählt ist unter Ethylenglycol, Propylenglycol, Glycerin, Pentaerythrit, Pentosen, Hexosen, Oxalsäure, Ammoniumoxalat, Malonsäure, Maleinsäure, Fumarsäure, Bernsteinsäure, Ascorbinsäure; Benzoesäure, o-, m- und p-Tolylsäure. Phthalsäure, Phthalsäureanhydrid, Isophthalsäure, Terephthalsäure, Dimethylformamid, Dimethylacetamid, N-Methylpyrrolidon.

10. Verfahren nach einem der vorhergehenden Ansprüche, wobei das Multimetalloxid die allgemeine Formel I aufweist
Ag_{a-c}Q_{b}M_{c}V₂O_{d} * e H₂O, I
worin
a einen Wert von 0,3 bis 1,9 hat,
Q für ein unter P, As, Sb und/oder BI ausgewähltes Element steht,
b einen Wert von 0 bis 0,3 hat,
M für wenigstens ein unter Alkali- und Erdalkalimetallen, Bi, Tl, Cu, Zn, Cd, Pb, Cr, Au, Al, Fe, Co, Ni, Mo, Nb, Ce, W, Mn, Ta, Pd, Pt, Ru und/oder Rh ist ausgewähltes Metall steht,
c einen Wert von 0 bis 0,5 hat, mit der Maßgabe, dass (a-c) ≥ 0,1 ist,
d eine Zahl, die sich durch die Wertigkeit und Häufigkeit der von Sauerstoff verschiedenen Elemente in der Formel I bestimmt, bedeutet und
e einen Wert von 0 bis 20 hat.

11. Verfahren nach Anspruch 10, wobei das Multimetalloxid in einer Kristallstruktur vorliegt, deren Pulverröntgendiagramm durch Beugungsreflexe bei den Netzebenenabständen d 15,23 ± 0,6, 12,16 ± 0,4, 10,68 ± 0,3, 3,41 ± 0,04, 3,09 ±0,04, 3,02 ± 0,04, 2,36 ± 0,04 und 1,80 ± 0,04 Å **gekennzeichnet** ist.

## Claims

1. A process for converting a precatalyst which comprises an inert support, an organic carbon source and a multimetal oxide comprising silver and vanadium to a gas phase oxidation catalyst which comprises the inert support and a catalytically active silver vanadium oxide bronze, in which the average vanadium oxidation state is from 4.5 to 4.7, the precatalyst initially comprising an amount of carbon source which is greater than a critical amount or corresponds to it, wherein
(i) the amount of carbon source is adjusted by burning-off to a value below the critical amount by treating the precatalyst in an oxygenous atmosphere at a temperature of from 80 to 200°C; and then
(ii) the precatalyst is treated thermally at a temperature of at least 350°C in a gas atmosphere which comprises less than 10% by volume of oxygen,
wherein the critical amount is defined as the amount of carbon source from which reduction to elemental silver occurs in the course of the thermal treatment of the precatalyst.

2. The process according to claim 1, wherein the thermal treatment is carried out in an inert gas stream.

3. The process according to claim 1 or 2, wherein, before the thermal treatment, the amount of the carbon source in the precatalyst is adjusted to a value in the range from 0.5 to less than 2% by weight, calculated as carbon and based on the weight of the multimetal oxide.

4. The process according to claim 3, wherein, before the thermal treatment, the amount of the carbon source in the precatalyst is adjusted to a value of less than or equal to 1.3% by weight.

5. The process according to any of the preceding claims, wherein the burning-off comprises at least one heating phase during which the temperature of the precatalyst is increased at a rate of less than 5°C/min and at least one plateau phase during which the temperature of the precatalyst is kept essentially constant.

6. The process according to any of the preceding claims, wherein the burning-off is carried out in an air stream.

7. The process according to any of the preceding claims, wherein the carbon source is selected from (i) compounds which have from 1 to 12 carbon atoms and at least one functional group which is selected from OH, C=O and NH₂; and (ii) polymeric compounds which are formed from repeat units which have from 2 to 12 carbon atoms and at least one functional group which is selected from OH, C=O and NH₂.

8. The process according to claim 7, wherein the carbon source is selected from compounds which have from 2 to 6 carbon atoms and at least two functional groups which are each independently selected from OH, C=O and NH₂.

9. The process according to claim 7, wherein the carbon source is selected from ethylene glycol, propylene glycol, glycerol, pentaerythritol, pentoses, hexoses, oxalic acid, ammonium oxalate, malonic acid, maleic acid, fumaric acid, succinic acid, ascorbic acid, benzoic acid, o-, m- and p-toluic acid, phthalic acid, phthalic anhydride, isophthalic acid, terephthalic acid, dimethylformamide, dimethylacetamide, N-methylpyrrolidone.

10. The process according to any of the preceding claims, wherein the multimetal oxide has the general formula I
Ag_{a-c}Q_{b}M_{c}V₂O_{d} * e H₂O I
where
a is from 0.3 to 1.9,
Q is an element selected from P, As, Sb and/or Bi,
b is from 0 to 0.3,
M is at least one metal selected from alkali metals and alkaline earth metals, Bi, Tl, Cu, Zn, Cd, Pb, Cr, Au, Al, Fe, Co, Ni, Mo, Nb, Ce, W, Mn, Ta, Pd, Pt, Ru and/or Rh,
c is from 0 to 0.5, with the proviso that (a-c) ≥ 0.1,
d is a number which is determined by the valency and frequency of the non- oxygen elements in the formula I, and
e is from 0 to 20.

11. The process according to claim 10, wherein the multimetal oxide is present in a crystal structure whose powder X-ray diagram is **characterized by** reflections at the interplanar spacings d of 15.23 ± 0.6, 12.16 ± 0.4, 10.68 ± 0.3, 3.41 ± 0.04, 3.09 ±0.04, 3.02 ± 0.04, 2.36 ± 0.04 and 1.80 ± 0.04 Å.

## Revendications

1. Procédé de transformation d'un précatalyseur, qui comprend un support inerte, une source de carbone organique et un oxyde de plusieurs métaux contenant de l'argent et du vanadium, en un catalyseur d'oxydation en phase gazeuse, qui comprend le support inerte et un bronze oxyde de vanadium-argent catalytiquement actif, dont le niveau d'oxydation moyen du vanadium est de 4,5 à 4,7, le précatalyseur contenant au début une quantité de source de carbone qui est supérieure à une quantité critique ou correspond à celle-ci, selon lequel
(i) la quantité de source de carbone est ajustée par combustion à une valeur inférieure à la quantité critique par traitement du précatalyseur dans une atmosphère contenant de l'oxygène à une température de 80 à 200 °C ; puis
(ii) le précatalyseur est traité thermiquement à une température d'au moins 350 °C dans une atmosphère gazeuse qui contient moins de 10 % en volume d'oxygène, la quantité critique étant définie comme la quantité de source de carbone à partir de laquelle une réduction en argent élémentaire a lieu lors du traitement thermique du précatalyseur.

2. Procédé selon la revendication 1, dans lequel le traitement thermique est réalisé dans un courant de gaz inerte.

3. Procédé selon la revendication 1 ou 2, dans lequel la quantité de la source de carbone dans le précatalyseur est ajustée avant le traitement thermique à une valeur dans la plage allant de 0,5 à moins de 2 % en poids, calculée en tant que carbone par rapport au poids de l'oxyde de plusieurs métaux.

4. Procédé selon la revendication 3, dans lequel la quantité de la source de carbone dans le précatalyseur est ajustée avant le traitement thermique à une valeur inférieure ou égale à 1,3 % en poids.

5. Procédé selon l'une quelconque des revendications précédentes, dans lequel la combustion comprend au moins une phase de chauffage, pendant laquelle la température du précatalyseur est augmentée à une vitesse inférieure à 5 °C/min, et au moins une phase de plateau, pendant laquelle la température du précatalyseur est maintenue sensiblement constante.

6. Procédé selon l'une quelconque des revendications précédentes, dans lequel la combustion est réalisée dans un courant d'air.

7. Procédé selon l'une quelconque des revendications précédentes, dans lequel la source de carbone est choisie parmi (i) les composés de 1 à 12 atomes de carbone qui comportent au moins un groupe fonctionnel choisi parmi OH, C=O et NH₂ ; et (ii) les composés polymères formés à partir d'unités de répétition de 2 à 12 atomes de carbone qui comportent au moins un groupe fonctionnel choisi parmi OH, C=O et NH₂.

8. Procédé selon la revendication 7, dans lequel la source de carbone est choisie parmi les composés de 2 à 6 atomes de carbone qui comportent au moins deux groupes fonctionnels choisis indépendamment l'un de l'autre parmi OH, C=O et NH₂.

9. Procédé selon la revendication 7, dans lequel la source de carbone est choisie parmi l'éthylène glycol, le propylène glycol, la glycérine, le pentaérythrite, le pentosène, l'hexosène, l'acide oxalique, l'oxalate d'ammonium, l'acide malonique, l'acide maléique, l'acide fumarique, l'acide succinique, l'acide ascorbique, l'acide benzoïque, l'acide o-, m- et p-tolylique, l'acide phtalique, l'anhydride de l'acide phtalique, l'acide isophtalique, l'acide téréphtalique, le diméthylformamide, le diméthylacétamide, la N-méthylpyrrolidone.

10. Procédé selon l'une quelconque des revendications précédentes, dans lequel l'oxyde de plusieurs métaux présente la formule générale I
Ag_{a-c}Q_{b}M_{c}V₂O_{d} * e H₂O I,
dans laquelle
a a une valeur de 0,3 à 1,9,
Q représente un élément choisi parmi P, As, Sb et/ou Bi,
b a une valeur de 0 à 0,3,
M représente un métal choisi parmi les métaux alcalins et alcalino-terreux, Bi, Tl, Cu, Zn, Cd, Pb, Cr, Au, Al, Fe, Co, Ni, Mo, Nb, Ce, W, Mn, Ta, Pd, Pt, Ru et/ou Rh,
c a une valeur de 0 à 0,5, à condition que (a-c) ≥ 0,1,
d signifie un nombre déterminé par la valence et la fréquence des éléments différents de l'oxygène dans la formule I, et
e a une valeur de 0 à 20.

11. Procédé selon la revendication 10, dans lequel l'oxyde de plusieurs métaux se présente selon une structure cristalline dont le diagramme de rayons X sur poudre est **caractérisé par** des réflexions de diffraction aux distances interplanaires d 15,23 ± 0,6, 12,16 ± 0,4, 10,68 ± 0,3, 3,41 ± 0,04, 3,09 ± 0,04, 3,02 ± 0,04, 2,36 ± 0,04 et 1,80 ± 0,04 Å.
